# EUROPEAN PATENT APPLICATION

(11) **EP 4 353 259 A1**
(43) Date of publication of application: **17.04.2024**
(21) Application number: 22811355.1
(22) Date of filing: 25.05.2022
(51) Int. Cl.: A61K 45/00, A61K 8/44, A61K 8/49, A61K 8/64, A61K 31/195, A61K 31/198, A61K 31/381, A61K 38/08, A61P 17/00, A61P 17/04, A61P 37/08, A61P 43/00, A61Q 19/00

(54) **AGENT FOR PREVENTING OR AMELIORATING PRURITUS**

(30) Priority: 26.05.2021 JP 2021088479; 30.11.2021 JP 2021194728
(71) Applicant: Kao Corporation, Tokyo 103-8210 (JP)
(72) Inventor: MATSUMOTO, Tomohiro, Haga-gun, Tochigi 321-3497 (JP); YOKOTA, Masafumi, Haga-gun, Tochigi 321-3497 (JP); ISHIKAWA, Junko, Haga-gun, Tochigi 321-3497 (JP); TOMINAGA, Mitsutoshi, Urayasu-shi, Chiba 279-0021 (JP); TAKAMORI, Kenji, Urayasu-shi, Chiba 279-0021 (JP)
(74) Representative: Vossius & Partner Patentanwälte Rechtsanwälte mbB
(86) International application number: PCT/JP2022/021408
(87) International publication number: WO 2022/250085

(57) **Abstract**

A novel target molecule for pruritus was found, and a novel means for eliminating pruritus is provided. An agent for preventing or ameliorating intractable pruritus, comprises a C3a receptor antagonist as an active ingredient.

## Description

### Field of the Invention

The present invention relates to an agent for preventing or ameliorating pruritus, particularly to an agent for preventing or ameliorating intractable pruritus, an agent for preventing or ameliorating a pruritic skin disease presenting with intractable pruritus, and a method for evaluating or selecting these agents.

### Background of the Invention

Pruritus is a clinical condition seen not only in skin diseases such as atopic dermatitis, but also in visceral diseases such as renal failure. Pruritus can also be triggered by dry skin, sunburn, and the rubbing of skin against clothing. The act of scratching due to pruritus physically injure the skin and cause further aggravation of the symptoms, and therefore the resolution of pruritus contributes to the prevention or amelioration of skin diseases. For example, it has been reported that skin symptoms caused by atopic dermatitis are prevented or ameliorated in mice whose hindlimb nails are cut to suppress physical damage to the skin caused by the act of scratching (Non Patent Literature 1).

The sensation of pruritus in peripheral tissues such as the skin is transmitted to the brain by afferent sensory nerves which connect the periphery to the dorsal horn of the spinal cord. The cell bodies of afferent sensory nerves reside in the dorsal root ganglia (DRG), and nerve fibers extend from the cell bodies to peripheral tissues and the dorsal horn of the spinal cord. Afferent sensory nerves are responsible for receiving sensations in the skin and transmitting them to secondary neurons in the dorsal horn of the spinal cord.

Pruritogenic substances cause pruritus by binding to the corresponding receptors. Histamine, serotonin, and chloroquine have been reported as pruritogenic substances, and Th2 cytokines such as IL-4 and IL-13 as pruritus-enhancing substances (sensitizers). Of these, a typical chemical pruritogenic substance is histamine, which is mainly secreted by mast cells. Recently, it is considered that histamine is essentially involved in only some acute pruritus, and its involvement in most of the diseases associated with chronic pruritus is poor (Non Patent Literature 2). Therefore, while antihistamines (H1 receptor antagonists) are currently often used as a drug to suppress pruritus, a sufficient therapeutic effect of antihistamines can be obtained against very limited pruritic conditions, and most pruritic conditions are intractable and difficult to remit with antihistamines. Antihistamines here refer to H1 receptor antagonists such as diphenhydramine. For example, the treatment of atopic dermatitis and xeroderma with antihistamines has been reported to be inadequate (Non Patent Literatures 3 and 4), and pruritus associated with many skin diseases such as atopic dermatitis and xeroderma, as well as internal diseases such as renal failure, is considered to be intractable. The mechanism of most cases of intractable pruritus has not been elucidated, and there is a need for the elucidation of the mechanism and the development of novel target molecules.

(Non Patent Literature 1) Hashimoto Y et al. Life Sciences. 2004 Dec 31;76(7) :783-94
(Non Patent Literature 2) Ikoma A et al. Nature Reviews Neuroscience. 2006 Jul;7(7) :535-47
(Non Patent Literature 3) J Am Acad Dermatol. 2014 Jul; 71(1): 116-132
(Non Patent Literature 4) Future Oncol. 2018 Oct;14(24):2531-2541

### Summary of the Invention

The present invention relates to the following 1) to 4) :
1) An agent for preventing or ameliorating intractable pruritus, comprising a C3a receptor antagonist as an active ingredient.
2) An agent for preventing or ameliorating a pruritic skin disease presenting with intractable pruritus, comprising a C3a receptor antagonist as an active ingredient.
3) A method for evaluating or selecting an agent for preventing or ameliorating intractable pruritus, comprising the following steps:
   (1) a step of evaluating the antagonistic effect of a test substance on C3a receptors, and
   (2) a step of evaluating or selecting the test substance having an antagonistic effect on C3a receptors as an agent for preventing or ameliorating intractable pruritus.
4) A method for evaluating or selecting an agent for preventing or ameliorating a pruritic skin disease presenting with intractable pruritus, comprising the following steps:
   (1) a step of evaluating the antagonistic effect of a test substance on C3a receptors, and
   (2) a step of evaluating or selecting the test substance having an antagonistic effect on C3a receptors as an agent for preventing or ameliorating a pruritic skin disease presenting with intractable pruritus.

### Brief Description of Drawings

Figure 1 shows the results of an RNA-seq analysis of AEW model DRG tissue.
Figure 2 shows the results of real-time PCR analysis of Vgf expression in AEW model.
Figure 3 shows the results of real-time PCR analysis of Vgf expression level in AD model.
Figure 4 shows the scratching frequency in C57BL/6J mice after TLQP-21 administration.
Figure 5 shows the scratching frequency in mast cell-deficient mice after TLQP-21 administration.
Figure 6 shows the C3a receptor antagonistic effect of Compound 1 and Compound 2.
Figure 7 shows the ameliorating effect on pruritus in an AD mouse model by the administration of a C3a receptor antagonist.
Figure 8 shows the ameliorating effect on pruritus in an AEW mouse model by the administration of a C3a receptor antagonist.

### Detailed Description of the Invention

The present invention relates to a finding of a novel target molecule for intractable pruritus, and a provision of a novel means for eliminating intractable pruritus.

The present inventors first collected dorsal root ganglia (DRG) from model mice showing xeroderma-like skin symptoms by treatment with an acetone-ether mixture and water (hereinafter referred to as AEW model mice) and performed a comprehensive gene expression analysis, which identified Vgf as a gene showing differential expression. Furthermore, when a quantitative analysis of the Vgf gene expression in DRG of AEW model mice and atopic dermatitis model mice (hereinafter referred to as AD model mice) was performed, Vgf expression in DRG tissue was significantly increased in both model mice compared to control mice. Vgf is the gene encoding the neurosecretory factor, VGF nerve growth factor inducible (hereinafter referred to as VGF). VGF is known to undergo degradation by proteases and the like in the organism, which produces several bioactive peptides (Lewis JE et al. Front Endocrinol (Lausanne). 2015 Feb 2;6:3). The injection of one of them, TLQP-21, into the skin of the posterior neck of healthy mice triggered scratching behavior in the mice, and thus TLQP-21 was found to induce pruritus. This scratching behavior induced by intradermal TLQP-21 administration was also observed in mast cell-deficient mice, suggesting that TLQP-21 induction of pruritus is a mast cell-independent response.

TLQP-21 is known to bind to and elicit signals from the complement factor C3a receptor, which is a GPCR receptor located on the cell membrane (Cero C et al Structure. 2014 Dec 2;22(12):1744-1753). In the process of preparing AD and AEW model mice, continuous administration in parallel of a compound which antagonizes the C3a receptor to mice was confirmed to significantly reduce scratching behavior, and thereby the C3a receptor was found to be a target for suppressing intractable pruritus. The present invention is based on these findings.

The present invention provides a novel agent which can be used to prevent or ameliorate intractable pruritus and a pruritic skin disease presenting with intractable pruritus.

The active ingredient of the agent for preventing or ameliorating intractable pruritus and agent for preventing or ameliorating a pruritic skin disease presenting with intractable pruritus of the present invention is a C3a receptor antagonist. Complement factor C3a is an anaphylatoxin which acts on mast cells, basophils and the like, and elicit various biological responses. In addition to C3a produced upon complement system activation, the C3a receptor also accepts TLQP-21. It has been reported that the inhibition of C3a receptors suppresses cerebral edema and cerebral hemorrhage induced by stroke (PLoS One. 2017 Jul 10;12(7):e0180822) and reduces IgG-induced arthritis (J Pharmacol Sci. 2010;112(1):56-63), but its action on intractable pruritus is completely unknown.

In the present description, the "C3a receptor antagonist" may be a competitive antagonist or a non-competitive antagonist. C3a receptor antagonists include low molecular weight compounds, aptamers composed of oligonucleotides, peptides and the like, and biological preparations such as neutralizing antibodies. In the present description, it may also be an inhibitor of C3a receptor expression.

Examples of C3a receptor antagonists which are low molecular weight compounds include compounds of the following formula (1) or compounds of the following formula (2): 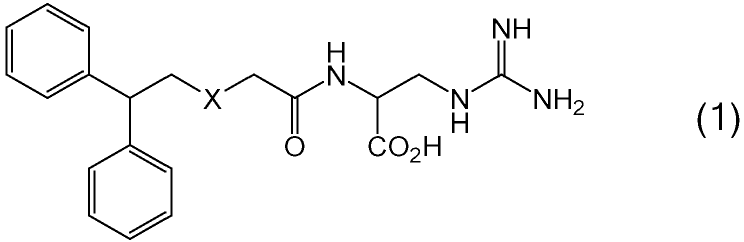 wherein X is O, S, CH₂ or NH, 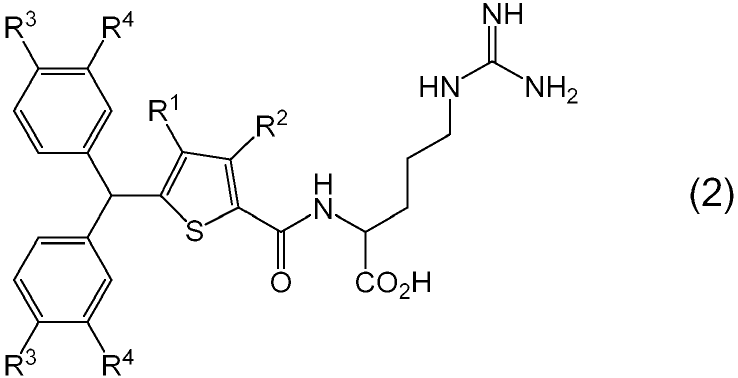 wherein R¹ is a hydrogen atom, a methyl group or a phenyl group, R² is a hydrogen atom, a methyl group or a bromine atom, R³ is a hydrogen atom, a hydroxy group, a methoxy group, a fluorine atom, or a chlorine atom, and R⁴ is a hydrogen atom or a chlorine atom.

Preferred specific examples of the compounds of formula (1) include SB290157 of the following structural formula wherein X is O, and (2S)-5-(diaminomethylideneamino)-2-[[2-(2,2-diphenylethylsulfanyl)acetyl]amino]pentanoic acid wherein X is S (Reid RC. et al. Journal of Medicinal Chemistry. 2014 Oct 23;57(20):8459-70.).

On the other hand, preferred specific examples of the compounds of formula (2) include Compound 1 of the following structural formula, wherein R¹, R², R³ and R⁴ are all hydrogen atoms (L-arginine, N2-[[5-(diphenylmethyl)-2-thienyl]carbonyl]), and Compound 2 of the following structural formula, wherein R¹ is a hydrogen atom, R² is a methyl group, R³ is a chlorine atom and R⁴ is a hydrogen atom (L-arginine, N2-[[5-[bis(4-chlorophenyl)methyl]-3-methyl-2-thienyl]carbonyl]) (Rowley, J. A. et al. Journal of Medicinal Chemistry, 2020; 63 (2) :529-541) .

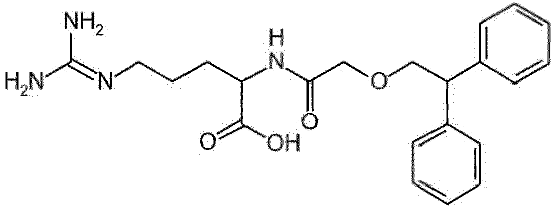

### (SB290157)

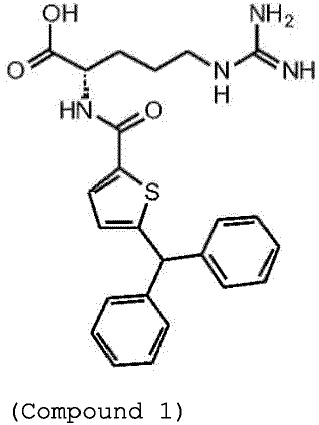

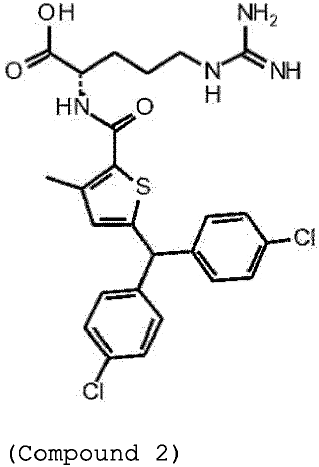

Furthermore, the present invention also includes hexapeptides with partial common sequences, represented by the amino acid sequence FLTChaAR (Cha: cyclohexylalanine), as C3a receptor antagonists (Conor C G Scully. et al. Journal of Medicinal Chemistry. 2010 8;53 (13) :4938-48) .

The above compounds may be salts, solvates or non-solvates, and are all included.

These are known compounds, which can be obtained by chemical synthesis based on previous reports (e.g., Rowley, J. A. et al. Journal of Medicinal Chemistry, 2020;63(2):529-541). They may also be commercially obtained.

C3a receptor expression inhibitors include antisense oligonucleotides, siRNA, miRNA, and ribozymes. These expression inhibitors can be modifications or derivatives such as phosphorothioate modifications. These expression inhibitors bind to DNA or mRNA encoding the C3a receptor, which inhibits their transcription or translation, or promotes mRNA degradation, thereby suppressing protein expression.

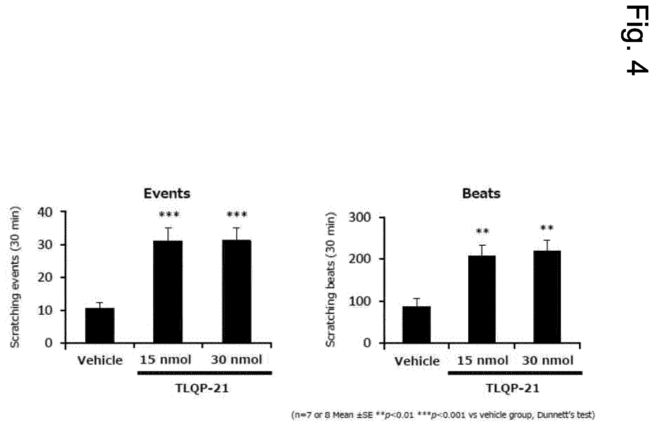

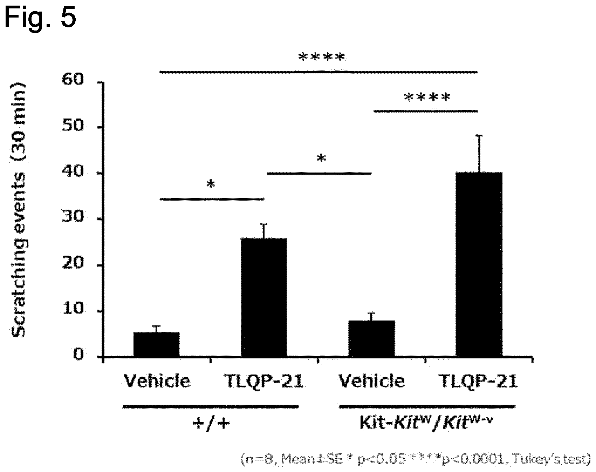

As shown in the examples below, the injection of TLQP-21, which is produced by the degradation of VGF, into the skin of the posterior neck of healthy mice triggered scratching behavior in the mice (Figure 4). This result clearly shows that TLQP-21 induces pruritus. The scratching behavior induced by intradermal TLQP-21 administration was also observed in mast cell-deficient mice (Figure 5), which suggests that TLQP-21 induction of pruritus is a mast cell-independent response.

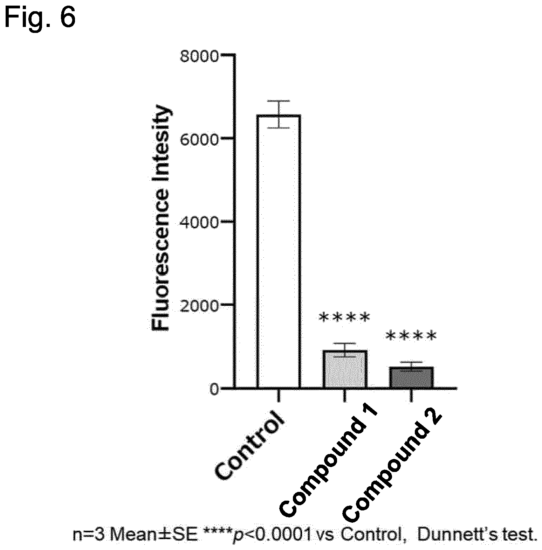

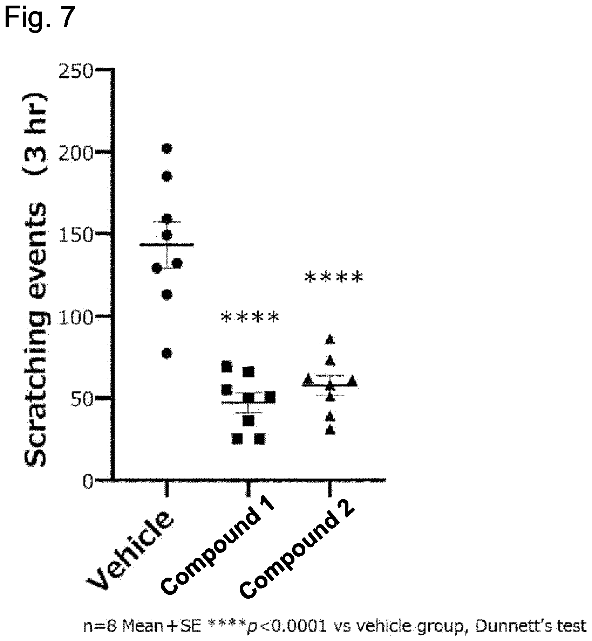

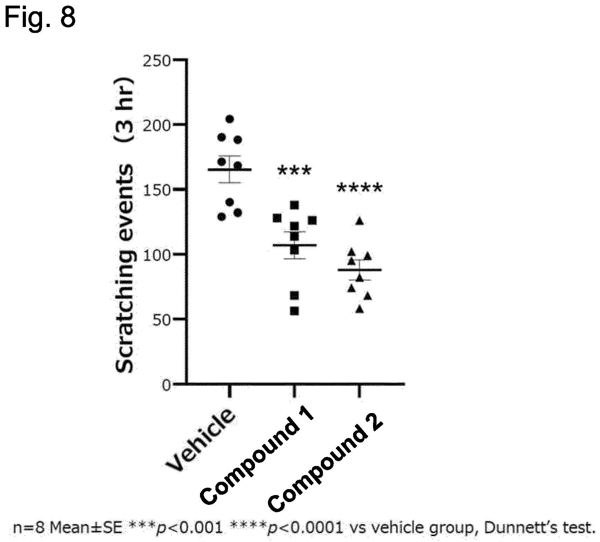

As described above, TLQP-21 is known to bind to and elicit signals from C3a receptors. Furthermore, when Compound 1 or Compound 2, which are known to antagonize the receptor of C3a and was confirmed to have C3a receptor antagonism (Figure 6), were continuously administered to mice in the process of preparing AD and AEW model mice, a significant decrease in scratching behavior was observed in the model mice administered with Compound 1 or Compound 2 (Figures 7 and 8). This reduction in scratching behavior indicates soothing of pruritus. This clearly shows that C3a receptors are a target for suppressing intractable pruritus. Therefore, C3a receptor antagonists have the effect of suppressing intractable pruritus, and can be agents for preventing or ameliorating intractable pruritus, and agents for preventing or ameliorating a pruritic skin disease presenting with intractable pruritus (hereinafter also referred to as "agents for preventing or ameliorating intractable pruritus and the like"), or can be used to produce these agents. C3a receptor antagonists can also be applied to animals, including humans, and can be used to prevent or ameliorate intractable pruritus, and to prevent or ameliorate a pruritic skin disease presenting with intractable pruritus. Furthermore, it is possible to search for agents for preventing or ameliorating intractable pruritus and agents for preventing or ameliorating a pruritic skin disease presenting with intractable pruritus by using C3a receptor antagonism as an indicator.

Similarly, the C3a receptor was found to be a target for suppressing pruritus which does not involve mast cells. Therefore, C3a receptor antagonists have the effect of suppressing pruritus in which mast cells are not involved, and can be agents for preventing or ameliorating pruritus in which mast cells are not involved, and agents for preventing or ameliorating a pruritic skin disease presenting with pruritus in which mast cells are involved (hereinafter also referred to as "agents for preventing or ameliorating pruritus in which mast cells are not involved and the like"), or can be used to produce these agents. C3a receptor antagonists can also be applied to animals, including humans, to prevent or ameliorate pruritus in which mast cells are not involved, and to prevent or ameliorate a pruritic skin disease presenting with pruritus in which mast cells are not involved. Furthermore, it is possible to search for agents for preventing or ameliorating pruritus in which mast cells are involved and agents for preventing or ameliorating a pruritic skin disease presenting with pruritus in which mast cells are not involved by using C3a receptor antagonism as an indicator.

Here, "use" can be use in humans or non-human animals, and may be therapeutic or non-therapeutic. "Non-therapeutic" is a concept which does not include medical practices, i.e., a concept which does not include methods of performing surgeries, treatment, or diagnosis on humans, and more specifically, a concept which does not include methods in which a physician or a person directed by a physician performs surgery, treatment, or diagnosis on humans.

In the present description, "pruritus" is a subjective sensation and its cause is not particularly limited. Examples of the site of pruritus include broad areas and specific sites, such as the entire body, scalp, face, back, arms, back of the hands, fingers, and legs.

The present invention is suitable for the prevention or amelioration of intractable pruritus.

"Intractable pruritus" means pruritus which is difficult to remit with antihistamines (H1 receptor antagonists), or pruritus which does not resolve. Examples of intractable pruritus include pruritus associated with skin diseases such as atopic dermatitis, contact dermatitis, asteatotic eczema, seborrheic dermatitis, nummular eczema, dermal pruritus, xeroderma, psoriasis, prurigo nodularis, and chronic prurigo, pruritus associated with autoimmune diseases such as pemphigoid and dermatomyositis, pruritus associated with renal failure, liver disease, diabetes, and other internal and endocrine diseases, pruritus associated with malignant lymphomas and neoplasms, and pruritus associated with neurological disorders.

"Pruritic skin disease" means a skin disease associated with pruritus. Examples thereof include urticaria, atopic dermatitis, contact dermatitis, asteatotic eczema, seborrheic dermatitis, nummular eczema, dermal pruritus, xeroderma, psoriasis, prurigo nodularis, chronic prurigo, pemphigoid and dermatomyositis. Of these, with the exception of urticaria, the skin diseases are pruritic skin diseases presenting with intractable pruritus. The present invention is suitable for pruritic skin diseases presenting with intractable pruritus.

"Prevention" refers to the prevention or delaying of the onset of symptoms in an individual, or the reduction of the risk of onset of symptoms in an individual.

"Amelioration" refers to the favorable change in a symptom or condition, the prevention or delaying of the aggravation of a symptom or condition, or the reversal, prevention, or delaying of the progression of a symptom.

The agent for preventing or ameliorating intractable pruritus and the like, and the agent for preventing or ameliorating pruritus in which mast cells are not involved and the like of the present invention may themselves be a medicament, a quasi-drug or a cosmetic for preventing or ameliorating intractable pruritus, for preventing or ameliorating a pruritic skin disease presenting with intractable pruritus, for preventing or ameliorating pruritus in which mast cells are not involved, or for preventing or ameliorating a pruritic skin disease presenting with pruritus in which mast cells are not involved, or may be a material or active ingredient contained in the medicament, quasi-drug, or cosmetic.

The medicament (including quasi-drugs, the same applies hereinafter) contains a C3a receptor antagonist as an active ingredient for preventing or ameliorating intractable pruritus, for preventing or ameliorating a pruritic skin disease presenting with intractable pruritus, for preventing or ameliorating pruritus in which mast cells are not involved, or for preventing or ameliorating a pruritic skin disease presenting with pruritus in which mast cells are not involved. Furthermore, the medicament may contain a pharmaceutically acceptable carrier, other active or pharmaceutical ingredients, or the like, as needed, as long as the function of the active ingredient is not lost.

The administration of the medicament containing a C3a receptor antagonist can be of any form, and examples thereof include oral and parenteral administration. Examples of dosage forms for oral administration include tablets, capsules, granules, powders, and syrups. Examples of dosage forms for parenteral administration include various preparations such as external skin, transdermal, transmucosal, transnasal, enteral, injectable, suppository, inhaled, and adhesive preparations. For parenteral administration, a suitable form of preparation is an external skin preparation, and specific examples thereof include in the form of an ointment, emulsified liquid, cream, emulsion, lotion, gel, and aerosol.

The cosmetic contains a C3a receptor antagonist as an active ingredient for preventing or ameliorating intractable pruritus, for preventing or ameliorating a pruritic skin disease presenting with intractable pruritus, for preventing or ameliorating pruritus in which mast cells are not involved, or for preventing or ameliorating a pruritic skin disease presenting with pruritus in which mast cells are not involved. Furthermore, the cosmetic may contain a cosmetically acceptable carrier, other active or cosmetic ingredients, or the like, as needed, as long as the function of the active ingredient is not lost.

Preferred examples of cosmetics containing a C3a receptor antagonist include cosmetics for the face and body (e.g., lotions, gels, creams, and facial masks), makeup cosmetics, and cleansers for the face and body.

Each of these medicament and cosmetic preparations can be produced according to a conventional method by combining a C3a receptor antagonist with a pharmaceutically or cosmetically acceptable carrier, the other active, pharmaceutical or cosmetic ingredients described above, and the like, as needed.

Examples of the pharmaceutically or cosmetically acceptable carrier include various oils, surfactants, gelling agents, buffers, preservatives, antioxidants, solvents, dispersants, chelating agents, thickeners, ultraviolet absorbers, emulsion stabilizers, pH adjusters, pigments, and fragrances.

Examples of the other active, pharmaceutical or cosmetic ingredients include plant extracts, fungicides, moisturizers, anti-inflammatory agents, antibacterial agents, keratolytic agents, refrigerants, antiseborrheic agents, cleansing agents, and makeup ingredients.

The content of the C3a receptor antagonist in the above medicament or cosmetic preparation cannot be generally defined, as it differs depending on the type of C3a receptor antagonist and the form of the preparation, but for example, based on the total amount of the preparation, it is preferably 0.001 mass% or more, more preferably 0.005 mass% or more, and preferably 0.1 mass% or less, more preferably 0.05 mass% or less. In addition, it is preferably from 0.001 to 0.1 mass%, and more preferably from 0.005 to 0.05 mass%.

The dosage or amount of C3a receptor antagonist used can be an amount which achieves the effect of the present invention. The dosage or amount used can vary according to the type of C3a receptor antagonist, the species, weight, sex, age, condition of the subject, or other factors, but in the case of parenteral administration such as of an external skin preparation, the dosage per adult (60 kg) is preferably 0.1 mg or more, more preferably 1 mg or more, and preferably 1 000 mg or less, more preferably 100 mg or less. In addition, it is preferably from 0.1 mg to 1 000 mg, and more preferably from 1 mg to 100 mg. In the present invention, such amounts can be administered or used repeatedly or continuously for 1 day or more, preferably 7 days or more, more preferably 14 days or more, and even more preferably 42 days or more, divided in one to several doses per day.

Examples of the subject of administration or use of the agent for preventing or ameliorating intractable pruritus and the like and the agent for preventing or ameliorating pruritus in which mast cells are not involved and the like of the present invention include humans or non-human animals requiring or desiring the prevention or amelioration of intractable pruritus, the prevention or amelioration of a pruritic skin disease presenting with intractable pruritus, the prevention or amelioration of pruritus in which mast cells are not involved, and the prevention or amelioration of a pruritic skin disease presenting with pruritus in which mast cells are not involved. Specific examples include humans having intractable pruritus or suffering from a pruritic skin disease presenting with intractable pruritus, and humans and non-human animals having pruritus in which mast cells are not involved, or suffering from a pruritic skin disease presenting with pruritus in which mast cells are not involved. Examples of non-human animals include non-human mammals such as anthropoid apes, other primates, and carnivore animals.

The site of administration or use of the agent for preventing or ameliorating intractable pruritus and the like and the agent for preventing or ameliorating pruritus in which mast cells are not involved and the like of the present invention is not particularly limited as long as it is a site where pruritus is felt.

The method for evaluating or selecting an agent for preventing or ameliorating intractable pruritus of the present invention includes (1) a step of evaluating an antagonistic effect of a test substance on C3a receptors, and (2) a step of evaluating or selecting the test substance having an antagonistic effect on C3a receptors as an agent for preventing or ameliorating intractable pruritus.

The method for evaluating or selecting an agent for preventing or ameliorating pruritic skin diseases presenting with intractable pruritus includes (1) a step of evaluating an antagonistic effect of a test substance on C3a receptors, and (2) a step of evaluating or selecting a test substance having an antagonistic effect on C3a receptors as an agent for preventing or ameliorating pruritic skin diseases presenting with intractable pruritus.

The method for evaluating or selecting an agent for preventing or ameliorating pruritus in which mast cells are not involved includes (1) a step of evaluating an antagonistic effect of a test substance on C3a receptors, and (2) a step of evaluating or selecting the test substance having an antagonistic effect on C3a receptors as an agent for preventing or ameliorating pruritus in which mast cells are not involved.

Furthermore, the method for evaluating or selecting an agent for preventing or ameliorating a pruritic skin disease presenting with pruritus in which mast cells are not involved includes (1) a step of evaluating an antagonistic effect of a test substance on C3a receptors, and (2) a step of evaluating or selecting the test substance having an antagonistic effect on C3a receptors as an agent for preventing or ameliorating a pruritic skin disease presenting with pruritus which does not involve mast cells.

Examples of the method for evaluating the antagonistic effect of a test substance on C3a receptors include the following method.

A test substance is brought into contact with cells having C3a receptors. A C3a receptor agonist can then be administered into the cells and evaluated by measuring the activity expressed by the binding of the agonist to the C3a receptor.

Examples of cells having C3a receptors include leukocyte cells such as mast cells and epithelial cells such as epidermal cells. The cells may be natural cells, genetically engineered recombinant cells, or cultures thereof.

The contact of the test substance with the cells having C3a receptors takes place in a culture medium or buffer solution, and any known medium or buffer solution can be used.

Any known C3a receptor agonist can be used, and examples thereof include TLQP-21, C3a, and α-cyclohexyl-N-[1-[1-oxo-3-(3-pyridinyl)propyl]-4-piperidinyl]-benzeneacetamide.

Any know method in the art can be used to measure the activity expressed by the binding of the C3a receptor agonist to the C3a receptor, with examples thereof including calcium imaging, cAMP assay, and TGFα shedding assay, and a test substance which suppresses this activity can be evaluated as having an antagonistic effect C3a receptors.

The evaluation of the antagonistic effect of a test substance on C3a receptors can be performed, for example, by the following method.

For example, the antagonistic effect on C3a receptors can be compared between a group with a higher concentration of the test substance and a group with a lower concentration of the test substance; between a test substance addition group and a placebo addition group; or before and after addition of the test substance, using ion influx, intracellular signaling, or the like associated with the activation of C3a receptors as an indicator.

The antagonistic effect of a test substance on C3a receptor scan be expressed by its IC50 value (concentration of test substance which inhibits C3a receptor activity by 50%), and as recognized in the art, a lower IC50 value indicates a higher inhibitory activity. The IC50 value of the test substance is preferably 1.0 µM or less, more preferably 0.1 µM or less, and even more preferably 0.01 µM.

If an antagonistic effect on C3a receptors is observed, the test substance can be evaluated or selected as an agent for preventing or ameliorating intractable pruritus or a pruritic skin disease presenting with intractable pruritus, or as an agent for preventing or ameliorating pruritus in which mast cells are not involved or a pruritic skin disease presenting with pruritus which does not involve mast cells.

The test substance is not particularly limited, and may be a naturally occurring substance or a substance artificially synthesized by chemical or biological methods or the like, and a compound, a composition or a mixture.

The test substance evaluated or selected as an agent for preventing or ameliorating intractable pruritus or a pruritic skin disease presenting with intractable pruritus, or as an agent for preventing or ameliorating pruritus in which mast cells are not involved or a pruritic skin disease presenting with pruritus in which mast cells are not involved, using the antagonistic effect on C3a receptors as an indicator, may be evaluated for its preventive or ameliorating effect on intractable pruritus, its preventive or ameliorating effect on pruritic skin diseases presenting with intractable pruritus, its preventive or ameliorating effect on pruritus in which mast cells are not involved, or its preventive or ameliorating effect on a pruritic skin disease presenting with pruritus in which mast cells are not involved, as needed.

In humans, subjective evaluation methods (visual analogue scale (VAS), numerical rating scale (NRS), verbal rating scale (VRS), 5D itch scale (5D), Shiratori pruritus severity criteria, and the like) and objective evaluation methods (video and audio recordings, sensor-based analysis, analysis by various wearable devices such as wristwatch-type devices, and the like) are useful for the evaluation of the preventive or ameliorating effect on intractable pruritus, the preventive or ameliorating effect on a pruritic skin disease presenting with intractable pruritus, the preventive or ameliorating effect on pruritus in which mast cells are not involved, or the preventive or ameliorating effect on a pruritic skin disease presenting with pruritus in which mast cells are not involved.

Specifically, for example, it is possible to evaluate these by measuring the scratching behavior of non-human animals administered with a substance inducing pruritus (various pruritus-inducing substances including C3a receptor agonists) and the test substance. It is also possible to evaluate these by measuring the scratching behavior when the test substance is administered to disease model animals which develop an intractable pruritic condition (various allergy models, congenital and acquired dermatitis-induced models, and the like).

Examples of non-human animals include mice, rats, hamsters, guinea pigs, rabbits, cats, dogs, and monkeys. The form of administration of the test substance to non-human animals may be either oral or parenteral, preferably intradermal administration, subcutaneous administration, application, intraperitoneal administration, or intravenous administration.

The scratching behavior of non-human animals can be observed by visual methods, moving image analysis such as videotaping, or by methods using measuring instruments such as MicroAct and SCLABA-Real.

Alternatively, humans having intractable pruritus or a pruritic skin disease presenting with intractable pruritus, or humans having pruritus in which mast cells are involved or a pruritic skin disease presenting with pruritus in which mast cells are not involved can be used as test subjects and administered the test substance to evaluate its preventive or ameliorating effect on intractable pruritus and a pruritic skin disease presenting with intractable pruritus, or its preventive or ameliorating effect on pruritus in which mast cells are not involved and a pruritic skin disease presenting with pruritus which does not involve mast cells.

In relation to the embodiments described above, the present invention further discloses the following aspects.
<1> An agent for preventing or ameliorating intractable pruritus, comprising a C3a receptor antagonist as an active ingredient.
<2> An agent for preventing or ameliorating a pruritic skin disease presenting with intractable pruritus, comprising a C3a receptor antagonist as an active ingredient.
<3> The agent according to <2>, wherein the pruritic skin disease presenting with intractable pruritus is preferably atopic dermatitis, contact dermatitis, asteatotic eczema, seborrheic dermatitis, nummular eczema, dermal pruritus, xeroderma, psoriasis, prurigo nodularis, chronic prurigo, pemphigoid or dermatomyositis, and more preferably atopic dermatitis or xeroderma.
<4> An agent for preventing or ameliorating pruritus in which mast cells are not involved, comprising a C3a receptor antagonist as an active ingredient.
<5> An agent for preventing or ameliorating a pruritic skin disease presenting with pruritus in which mast cells are not involved, comprising a C3a receptor antagonist as an active ingredient.
<6> The agent according to any of <1> to <5>, wherein the C3a receptor antagonist is preferably a compound of the following formula (1): 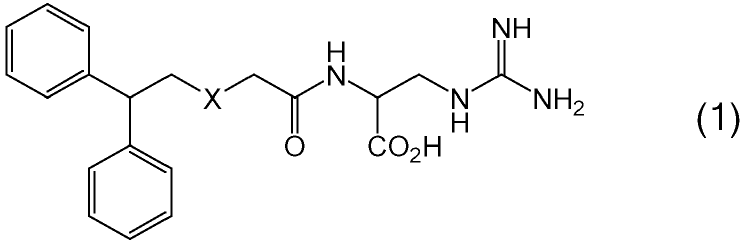 wherein X is O, S, CH₂ or NH,
   a compound of the following formula (2): 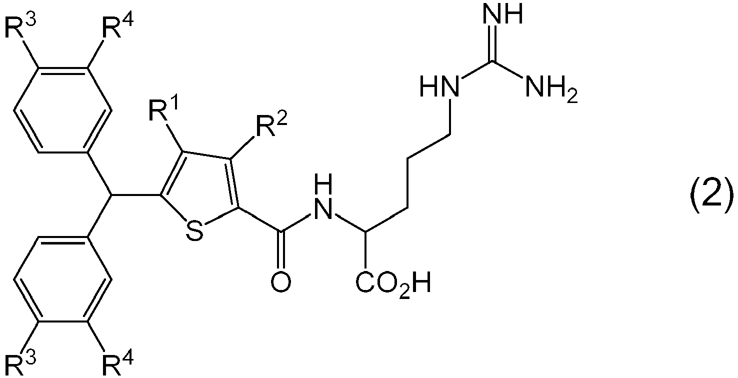 wherein R¹ is a hydrogen atom, a methyl group or a phenyl group, R² is a hydrogen atom, a methyl group or a bromine atom, R³ is a hydrogen atom, a hydroxy group, a methoxy group, a fluorine atom, or a chlorine atom, and R⁴ is a hydrogen atom or a chlorine atom
   or a hexapeptide consisting of the sequence FLTChaAR (Cha: cyclohexylalanine), more preferably SB290157, L-arginine, N2-[[5-(diphenylmethyl)-2-thienyl]carbonyl], L-arginine, N2-[[5-[bis(4-chlorophenyl)methyl]-3-methyl-2-thienyl]carbonyl], (2S)-5-(diaminomethylideneamino)-2-[[2-(2,2-diphenylethylsulfanyl)acetyl]amino]pentanoic acid or a hexapeptide consisting of the sequence FLTChaAR (Cha: cyclohexylalanine), and further more preferably L-arginine, N2-[[5-(diphenylmethyl)-2-thienyl]carbonyl] or L-arginine, N2-[[5-[bis(4-chlorophenyl)methyl]-3-methyl-2-thienyl]carbonyl].
<7> The agent according to any of <1> to <6>, which is blended as an active ingredient of a preparation.
<8> The agent according to any of <1> to <7>, wherein a content of the C3a receptor antagonist in the preparation is preferably 0.001 mass% or more, more preferably 0.005 mass% or more, preferably 0.1 mass% or less, more preferably 0.05 mass% or less, and preferably from 0.001 to 0.1 mass%, more preferably from 0.005 to 0.05 mass% based on the total amount of the preparation.
<9> The agent according to <7> or <8>, wherein the preparation is a medicament preparation.
<10> The agent according to any of <1> to <9>, wherein a dosage or amount of C3a receptor antagonist used is preferably 0.1 mg or more, more preferably 1 mg or more, preferably 1 000 mg or less, more preferably 100 mg or less, and preferably 0.1 mg to 1 000 mg, more preferably 1 mg to 100 mg per dose per adult (60 kg) in the case of parental administration.
<11> Use of a C3a receptor antagonist to produce an agent for preventing or ameliorating intractable pruritus.
<12> A C3a receptor antagonist for use in the prevention or amelioration of intractable pruritus.
<13> Non-therapeutic use of a C3a receptor antagonist for preventing or ameliorating intractable pruritus.
<14> Use of a C3a receptor antagonist for producing an agent for preventing or ameliorating pruritus in which mast cells are not involved.
<15> A C3a receptor antagonist for use in the prevention or amelioration of pruritus in which mast cells are not involved.
<16> Non-therapeutic use of a C3a receptor antagonist for preventing or ameliorating pruritus in which mast cells are not involved.
<17> Use of a C3a receptor antagonist for producing an agent for preventing or ameliorating a pruritic skin disease presenting with intractable pruritus.
<18> A C3a receptor antagonist for use in the prevention or amelioration of a pruritic skin disease presenting with intractable pruritus.
<19> Non-therapeutic use of a C3a receptor antagonist for preventing or ameliorating a pruritic skin disease presenting with intractable pruritus.
<20> The C3a receptor antagonist or use according to <17> to <19>, wherein the pruritic skin disease presenting with intractable pruritus is preferably atopic dermatitis, contact dermatitis, asteatotic eczema, seborrheic dermatitis, nummular eczema, dermal pruritus, xeroderma, psoriasis, prurigo nodularis, chronic prurigo, pemphigoid or dermatomyositis, and more preferably atopic dermatitis or xeroderma.
<21> Use of a C3a receptor antagonist for producing an agent for preventing or ameliorating a pruritic skin disease presenting with pruritus in which mast cells are not involved.
<22> A C3a receptor antagonist for use in the prevention or amelioration of a pruritic skin disease presenting with pruritus in which mast cells are not involved.
<23> Non-therapeutic use of a C3a receptor antagonist for preventing or ameliorating a pruritic skin disease presenting with pruritus in which mast cells are not involved.
<24> The C3a receptor antagonist or use according to any of <11> to <23>, wherein the C3a receptor antagonist is preferably a compound of the following formula (1): 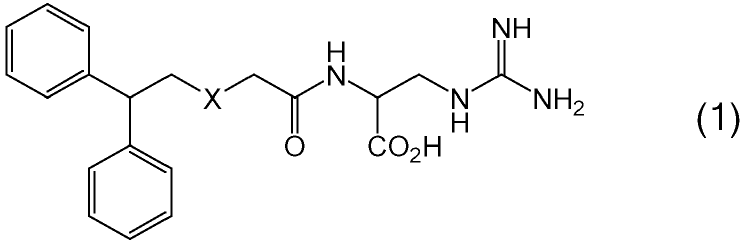 wherein X is O, S, CH₂ or NH,
   a compound of the following formula (2): 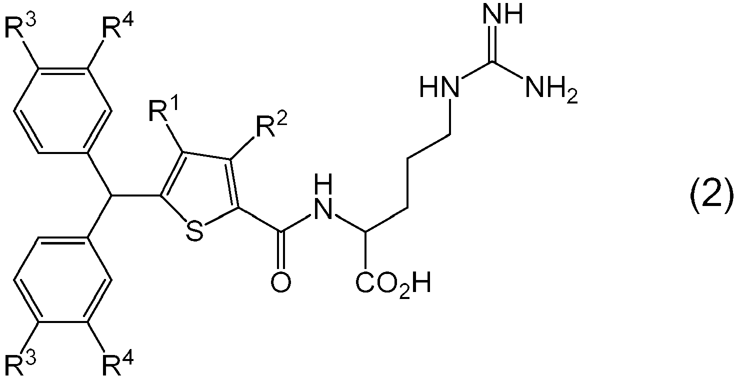 wherein R¹ is a hydrogen atom, a methyl group or a phenyl group, R² is a hydrogen atom, a methyl group or a bromine atom, R³ is a hydrogen atom, a hydroxy group, a methoxy group, a fluorine atom, or a chlorine atom, and R⁴ is a hydrogen atom or a chlorine atom
   or a hexapeptide consisting of the sequence FLTChaAR (Cha: cyclohexylalanine), more preferably SB290157, L-arginine, N2-[[5-(diphenylmethyl)-2-thienyl]carbonyl], L-arginine, N2-[[5-[bis(4-chlorophenyl)methyl]-3-methyl-2-thienyl]carbonyl], (2S)-5-(diaminomethylideneamino)-2-[[2-(2,2-diphenylethylsulfanyl)acetyl]amino]pentanoic acid or a hexapeptide consisting of the sequence FLTChaAR (Cha: cyclohexylalanine), and further more preferably L-arginine, N2-[[5-(diphenylmethyl)-2-thienyl]carbonyl] or L-arginine, N2-[[5-[bis(4-chlorophenyl)methyl]-3-methyl-2-thienyl]carbonyl].
<25> A method for preventing or ameliorating intractable pruritus, comprising administering or applying a C3a receptor antagonist to a subject.
<26> A method for preventing or ameliorating a pruritic skin disease presenting with intractable pruritus, comprising administering or applying a C3a receptor antagonist to a subject.
<27> The method according to <26>, wherein the pruritic skin disease presenting with intractable pruritus is preferably atopic dermatitis, contact dermatitis, asteatotic eczema, seborrheic dermatitis, nummular eczema, dermal pruritus, xeroderma, psoriasis, prurigo nodularis, chronic prurigo, pemphigoid or dermatomyositis, and more preferably atopic dermatitis or xeroderma.
<28> A method for preventing or ameliorating pruritus in which mast cells are not involved, comprising administering or applying a C3a receptor antagonist to a subject.
<29> A method for preventing or ameliorating a pruritic skin disease presenting with pruritus in which mast cells are not involved, comprising administering or applying a C3a receptor antagonist to a subject.
<30> The method according to any of <25> to <29>, wherein the C3a receptor antagonist is preferably a compound of the following formula (1): 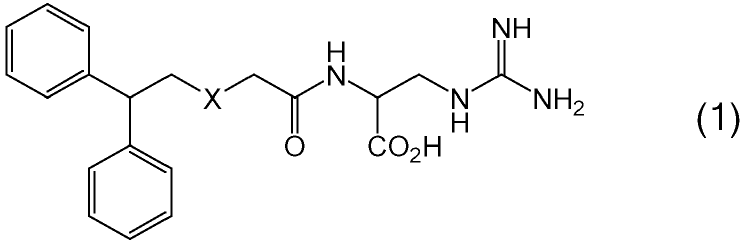 wherein X is O, S, CH₂ or NH,
   a compound of the following formula (2): 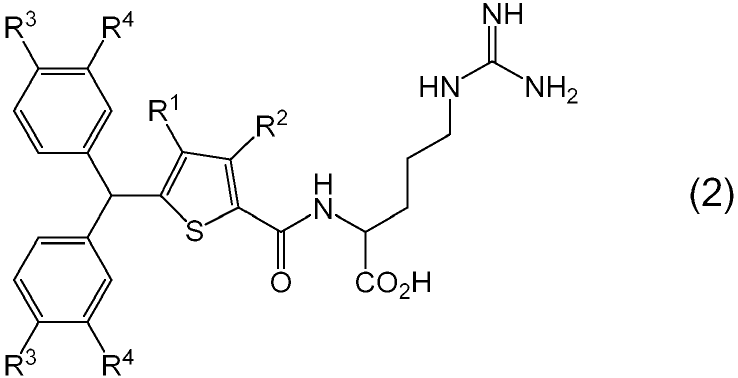 wherein R¹ is a hydrogen atom, a methyl group or a phenyl group, R² is a hydrogen atom, a methyl group or a bromine atom, R³ is a hydrogen atom, a hydroxy group, a methoxy group, a fluorine atom, or a chlorine atom, and R⁴ is a hydrogen atom or a chlorine atom
   or a hexapeptide consisting of the sequence FLTChaAR (Cha: cyclohexylalanine), more preferably SB290157, L-arginine, N2-[[5-(diphenylmethyl)-2-thienyl]carbonyl], L-arginine, N2-[[5-[bis(4-chlorophenyl)methyl]-3-methyl-2-thienyl]carbonyl], (2S)-5-(diaminomethylideneamino)-2-[[2-(2,2-diphenylethylsulfanyl)acetyl]amino]pentanoic acid or a hexapeptide consisting of the sequence FLTChaAR (Cha: cyclohexylalanine), and further more preferably L-arginine, N2-[[5-(diphenylmethyl)-2-thienyl]carbonyl] or L-arginine, N2-[[5-[bis(4-chlorophenyl)methyl]-3-methyl-2-thienyl]carbonyl].
<31> The method according to any of <25> to <30>, wherein a dosage or amount of C3a receptor antagonist applied is preferably 0.1 mg or more, more preferably 1 mg or more, preferably 1 000 mg or less, more preferably 100 mg or less, and preferably 0.1 mg to 1 000 mg, more preferably 1 mg to 100 mg per dose per adult (60 kg) in the case of parental administration.
<32> A method for evaluating or selecting an agent for preventing or ameliorating intractable pruritus, comprising the following steps:
   (1) evaluating an antagonistic effect of a test substance on C3a receptors, and
   (2) evaluating or selecting the test substance having an antagonistic effect on C3a receptors as an agent for preventing or ameliorating intractable pruritus.
<33> A method for evaluating or selecting an agent for preventing or ameliorating a pruritic skin disease presenting with intractable pruritus, comprising the following steps:
   (1) a step of evaluating an antagonistic effect of a test substance on C3a receptors, and
   (2) a step of evaluating or selecting the test substance having an antagonistic effect on C3a receptors as an agent for preventing or ameliorating a pruritic skin disease presenting with intractable pruritus.
<34> The method according to <32> or <33>, wherein the step (1) preferably comprises a step of bringing the test substance into contact with cells having C3a receptors, and then administering a C3a receptor agonist in the cells and measuring an activity expressed by binding of the agonist to the C3a receptor.
<35> The method according to <34>, wherein the cells having C3a receptors are preferably leukocyte cells or epithelial cells, and more preferably mast cells or epidermal cells.
<36> The method according to <34> or <35>, wherein the C3a receptor agonist is preferably TLQP-21, C3a, or α-cyclohexyl-N-[1-[1-oxo-3-(3-pyridinyl)propyl]-4-piperidinyl]-benzeneacetamide.
<37> The method according to any of <34> to <36>, wherein the measurement of the activity expressed by the binding of the C3a receptor agonist to the C3a receptor is preferably performed by calcium imaging, cAMP assay, and TGFα Shedding assay.
<38> The method according to any of <32> to <37>, further comprising after the step (2) a step of evaluating a preventive or ameliorating effect on intractable pruritus or a preventive or ameliorating effect on a pruritic skin disease presenting with intractable pruritus by measuring scratching behavior of non-human animals administered with the C3a receptor agonist and the test substance.
<39> The method according to any of <32> to <38>, further comprising after the step (2) a step of evaluating a preventive or ameliorating effect on intractable pruritus or the preventive or ameliorating effect on a pruritic skin disease presenting with intractable pruritus by administering the test substance to a human test subject having intractable pruritus or a pruritic skin disease presenting with intractable pruritus.
<40> A method for evaluating or selecting an agent for preventing or ameliorating pruritus in which mast cells are not involved, comprising the following steps:
   (1) evaluating an antagonistic effect of a test substance on C3a receptors, and
   (2) evaluating or selecting the test substance having an antagonistic effect on C3a receptors as an agent for preventing or ameliorating pruritus in which mast cells are not involved.
<41> A method for evaluating or selecting an agent for preventing or ameliorating a pruritic skin disease presenting with pruritus in which does mast cells are not involved, comprising the following steps:
   (1) evaluating an antagonistic effect of a test substance on C3a receptors, and
   (2) evaluating or selecting the test substance having an antagonistic effect on C3a receptors as an agent for preventing or ameliorating a pruritic skin disease presenting with pruritus in which mast cells are not involved.
<42> The method according to <40> or <41>, wherein the step (1) preferably comprises a step of bringing a test substance into contact with cells having C3a receptors, and then administering a C3a receptor agonist in the cells and measuring an activity expressed by binding of the agonist to the C3a receptor.
<43> The method according to <42>, wherein the cells having C3a receptors are preferably leukocyte cells or epithelial cells, and more preferably mast cells or epidermal cells.
<44> The method according to <42> or <43>, wherein the C3a receptor agonist is preferably TLQP-21, C3a, or α-cyclohexyl-N-[1-[1-oxo-3-(3-pyridinyl)propyl]-4-piperidinyl]-benzeneacetamide.
<45> The method according to any of <42> to <44>, wherein the measurement of the activity expressed by the binding of the C3a receptor agonist to the C3a receptor is preferably performed by calcium imaging, cAMP assay, and TGFα Shedding assay.
<46> The method according to any of <40> to <45>, further comprising after the step (2) a step of evaluating a preventive or ameliorating effect on pruritus in which mast cells are not involved or a preventive or ameliorating effect on a pruritic skin disease presenting with pruritus in which mast cells are not involved by measuring scratching behavior of non-human animals administered with the C3a receptor agonist and the test substance.
<47> The method according to any of <40> to <46>, further comprising after the step (2) a step of evaluating a preventive or ameliorating effect on pruritus in which mast cells are not involved or a preventive or ameliorating effect on a pruritic skin disease presenting with pruritus in which mast cells are not involved by administering the test substance to a human test subject having pruritus in which mast cells are not involved or a pruritic skin disease presenting with pruritus in which mast cells are not involved.

### Examples

### Example 1: Comprehensive Gene Expression Analysis

### 1. Experimental Animals

The animals used were male C57BL/6J mice and male NC/Nga mice.

Xeroderma model (AEW model): Absorbent cotton soaked with a 1:1 (v/v) mixture of acetone and diethyl ether was applied on the shaved skin of C57BL/6J mice and allowed to stand for 15 seconds. Immediately thereafter, absorbent cotton soaked with water was applied for 30 seconds. This treatment was repeated twice/day (morning and evening) for 7 days to prepare AEW model mice. Control mice on which only absorbent cotton soaked with water was applied for 30 seconds were prepared as a control. Each group n = 7 was used for the present analysis.

Atopic dermatitis model (AD model): NC/Nga mice were parasitized with mouse fur mites (M. musculi) to spontaneously develop dermatitis, and were used as an AD model. Mice of the same strain raised in mite-free SPF were used as control mice. Each group n = 8 was used for the present analysis.

### 2. Purification of total RNA

Dorsal root ganglia were excised from the cervical spine of mice. The excised tissues were homogenized by a polytron homogenizer, and total RNA was purified using QIAGEN's RNeasy Mini Kit.

### 3. Gene Expression Analysis

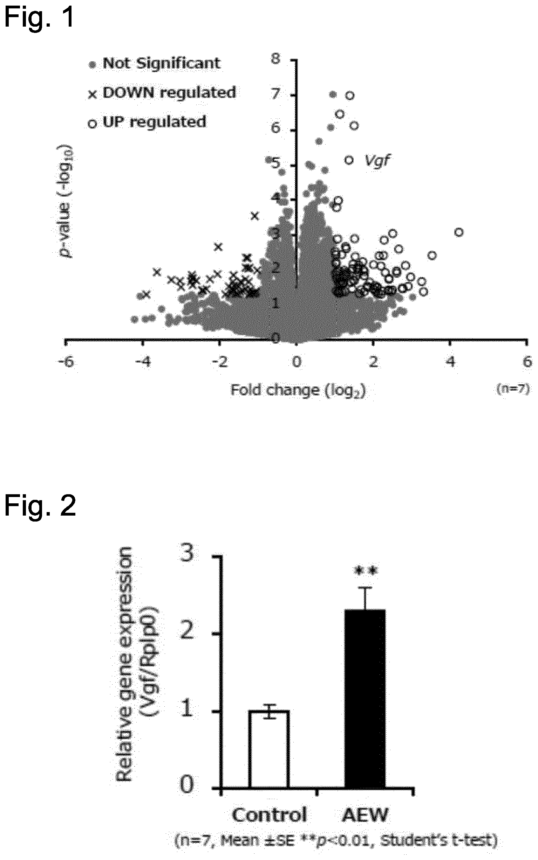

RNA-seq: Total RNA purified from AEW model mice and their control mice was used. SuperScript VILO (Thermo fisher scientific) was used for reverse transcription, and the subsequent treatments followed Thermo fisher scientific's Ion AmpliSeq standard protocol. Ion S5 system was used for sequencing. The results are shown in Figure 1. As shown in Figure 1, Vgf expression for a given sequencing volume was significantly increased in the AEW model, with p<0.05 by Student's t-test compared to control mice.

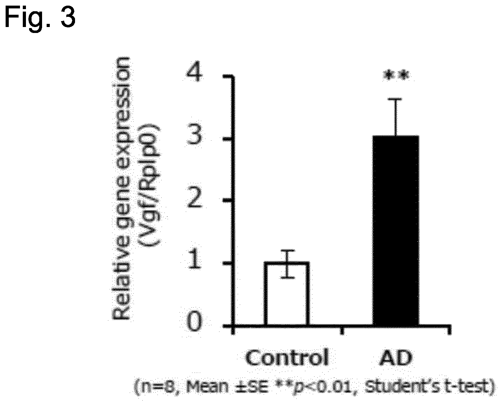

Real-Time PCR: Total RNAs purified from AEW model mice, AD model mice, and their control mice were used to perform quantitative PCR analysis of the Vgf gene by a Real-Time PCR System using TaqMan Gene Expression Assays specific for Vgf as the target gene. Rplp0 was used as the internal standard. Figures 2 and 3 show the relative expression levels of the Vgf gene when the Vgf gene expression level of control mice is set to 1.

As shown in Figure 2, the Vgf expression levels in DRG tissue were significantly increased in AEW model mice compared to control mice. As shown in Figure 3, the Vgf expression levels in DRG tissue were also significantly increased in AD model mice compared to control mice.

### Example 2: Expression of Pruritus by TLQP-21

### 1. Experimental Animals

The mice used were male C57BL/6J mice.

### 2. Administration of Candidate Mediator Molecule

Mouse TLQP-21 (Tocris Bioscience, sequence: TLQPPASSRRRHFHHALPPAR, the same applies hereinafter) was prepared at concentrations of 15 nmol/20 µL and 30 nmol/20 µL. Saline was used as the solvent. Twenty µL of this was administered into the shaved skin of the posterior neck of C57BL/6J mice. The measurement of the scratching behavior of the mice was started immediately after injection, and the values measured 30 minutes after the start were analyzed.

### 3. Measurement of Scratching Behavior

MicroAct (Neuroscience) was used to measure the scratching behavior after TLQP-21 administration. The results are shown in Figure 4. The total number of a series of continuous scratching behavior was indicated as Events, and the total number of scratching behavior throughout the measurement period was indicated as Beats.

As shown in Figure 4, after TLQP-21 administration, an increase in the mice's scratching behavior was observed as a significant increase in Events and Beats. The scratching behavior developed as an immediate response within 30 minutes.

### Example 3: TLQP-21-Dependent Expression of Pruritus in Mast Cell-Deficient Mice

### 1. Experimental Animals

WBB6F1/Kit-Kit^{W}/Kit^{W-v} mice were used as mast cell-deficient mice and WBB6F1+/+ mice were used as wild-type control mice. Male mice were used in both cases.

### 2. Administration of Mediator Molecule

Mouse TLQP-21 (Tocris Bioscience) was prepared at a concentration of 20 nmol/20 µL. Saline was used as the solvent. Twenty µL of this was administered into the shaved skin of the posterior neck of WBB6F1/Kit-Kit^{W}/Kit^{W-v} and WBB6F1+/+ mice. The measurement of the scratching behavior of the mice was started immediately after injection, and the values measured 30 minutes after the start were analyzed.

### 3. Measurement of Scratching Behavior

MicroAct (Neuroscience) was used to measure the scratching behavior after TLQP-21 administration. The results are shown in Figure 5. The total number of a series of continuous scratching behavior was indicated as Events.

As shown in Figure 5, an increase in scratching behavior due to TLQP-21 administration was observed in both mast cell-deficient and wild-type control mice.

### Example 4: Evaluation of C3a Receptor Antagonism

### 1. Evaluation Sample

Compound 1 or Compound 2 described above, synthesized in accordance with a previous report (Rowley, J. A. et al. Journal of Medicinal Chemistry, 2020;63(2):529-541) were used as C3a receptor antagonists. In the following evaluation, Compound 1 and Compound 2 were dissolved in DMSO and diluted with HBSS as appropriate.

### 2. Cells for Evaluating C3a Receptor Response

Human Embryonic Kidney cells 293 (HEK293) were used, transfected with the expression vectors necessary for evaluation according to the following.

A transfection solution was prepared by sequentially adding C3a receptor expression vector, GNA16 expression vector, and PEI-MAX (PolyScience) solution to serum-free DMEM medium, mixing well, and then allowing it to stand at room temperature for 20 minutes.

The prepared transfection solution was added to HEK293 cells seeded in a 6-well cell culture plate and the HEK293 cells were cultured for 18 hours or more to transfect them with the expression vectors. After culture, the medium was removed, and the cells were washed once with PBS, detached with 0.05% Trypsin/EDTA (Gibco) and subjected to the following Ca²⁺-flux assay.

### 3. Ca²⁺-Flux Assay

The cells for evaluating C3a receptor response described above were seeded in a 96-well Black Polystyrene Microplate (Thermo fisher scientific) precoated with Poly-L-Ornithine Solution (FUJIFILM Wako Pure Chemical). Calcium Kit-Fluo4 (DOJINDO) was used for the measurement of intracellular Ca²⁺ concentration during C3a receptor stimulation. The Ca²⁺ fluorescent indicator Fluo-4 was incorporated into the cells by adding a loading buffer prepared in accordance with the kit manual, and allowing it to stand at 37°C for 1 hour. After washing once with HBSS, a recording buffer with the C3a receptor antagonist, Compound 1 or Compound 2 dissolved therein was added. For the control, a recording buffer was added. After 15 minutes, a mouse TLQP-21 solution was added for C3a receptor stimulation, and the Ca²⁺ influx associated with the stimulation was measured over time as intracellular fluorescence intensity (Ex: 494 nm, Em: 525 nm). The value obtained by subtracting the measured value when HBSS was added instead of mouse TLQP-21 solution (blank) was used as the measured value. The C3a receptor antagonist solution and mouse TLQP-21 solution were added after adjusting their concentrations by appropriately diluting them so that their final concentrations when measuring fluorescence intensity were 10 nM and 1 µM, respectively. The addition of C3a receptor antagonist solution and TLQP-21 solution to the cells for evaluating C3a receptor response and the measurement of fluorescence intensity were performed using a fluorescent plate reader equipped with an automatic pipetting function. The integrated values of the fluorescence intensity measured over time for 3 min after the addition of mouse TLQP-21 solution are shown in Figure 6.

As shown in Figure 6, the fluorescence intensity due to C3a receptor stimulation by TLQP-21 measured in the control was greatly suppressed by the addition of Compound 1 or Compound 2, indicating that both of these compounds act as C3a receptor antagonists.

### Example 5: Amelioration of Pruritus in AD Model Mice by C3a Receptor Antagonist

### 1. Experimental Animals

Seven-week-old female NC/Nga mice were used. Preparation of AD model mice: The back of the neck was shaved, and 100 mg of an ointment containing mite allergen: Biostir AD (Biostir Inc.) was applied to the back of the neck and auricular region under isoflurane anesthesia for initial sensitization. Four days after the first application of the ointment containing mite allergen, 150 µL of 4% (w/v) SDS solution and 100 mg of ointment containing mite allergen were applied to the back of the neck and auricular region under isoflurane anesthesia. Thereafter, the same procedure was performed four times at a frequency of once every 3 to 4 days to induce AD-like dermatitis.

### 2. Sample Preparation and Method of Administration

Compound 1 or Compound 2, whose C3a receptor antagonism was confirmed in Example 4 above, was used as a C3a receptor antagonist. Compound 1 or Compound 2 was dissolved in saline containing 20% polyethylene glycol 400, of which 200 µL was administered subcutaneously to the back of the neck. The control group was administered with the same amount of only the above solvent. The concentration of the C3a receptor antagonist solution was set at 0.5 mg/mL for Compound 1 and 0.05 mg/mL for Compound 2. Sample administration started on the day of the second application of the ointment containing mite allergen and was performed three times a week.

### 3. Measurement of Scratching Behavior

Four days after the last application of the ointment containing mite allergen, the scratching behavior was measured for 3 hours after the last administration of the sample. MicroAct (Neuroscience) was used to measure the scratching behavior. The results are shown in Figure 7.

As shown in Figure 7, in AD model mice administered with Compound 1 or Compound 2, whose C3a receptor antagonism was confirmed, in parallel with the application of mite allergen, a significant decrease in scratching behavior was observed compared to AD model mice administered with solvent alone.

### Example 6: Amelioration of Pruritus in AEW Model Mice by C3a Receptor Antagonist

### 1. Experimental Animals

The animals used were male C57BL/6J mice. Preparation of AEW model: The back of the neck was shaved, and absorbent cotton soaked with a 1:1 (v/v) mixture of acetone and diethyl ether was applied and allowed to stand for 15 seconds. Immediately thereafter, absorbent cotton soaked with water was applied for 30 seconds. This treatment was repeated twice/day (morning and evening) for 7 days.

### 2. Sample Preparation and Method of Administration

Compound 1 or Compound 2, whose C3a receptor antagonism was confirmed in Example 4 above, was used as a C3a receptor antagonist. Compound 1 or Compound 2 was dissolved in saline containing 20% polyethylene glycol 400, of which 200 µL was administered subcutaneously to the back of the neck. The control group was administered with the same amount of only the above solvent. The concentration of the C3a receptor antagonist solution was set at 0.5 mg/mL for Compound 1 and 0.05 mg/mL for Compound 2. Sample administration started after the first AEW treatment and was performed at a frequency of once daily for 8 days.

### 3. Measurement of Scratching Behavior

Sixteen hours after the last AEW treatment, the scratching behavior was measured for 3 hours after the last administration of the sample. MicroAct (Neuroscience) was used to measure the scratching behavior. The results are shown in Figure 8.

As shown in Figure 8, in AEW model mice administered with Compound 1 or Compound 2, whose C3a receptor antagonism was confirmed, in parallel with AEW treatment, a significant decrease in scratching behavior was observed compared to AEW model mice administered with solvent alone.

## Claims

1. An agent for preventing or ameliorating intractable pruritus, comprising a C3a receptor antagonist as an active ingredient.

2. An agent for preventing or ameliorating a pruritic skin disease presenting with intractable pruritus, comprising a C3a receptor antagonist as an active ingredient.

3. The agent for preventing or ameliorating a pruritic skin disease according to claim 2, wherein the pruritic skin disease presenting with intractable pruritus is atopic dermatitis or xeroderma.

4. The agent according to any one of claims 1 to 3, wherein the C3a receptor antagonist is a compound of the following formula (1), a compound of the following formula (2), or a hexapeptide consisting of the sequence FLTChaAR (Cha: cyclohexylalanine), 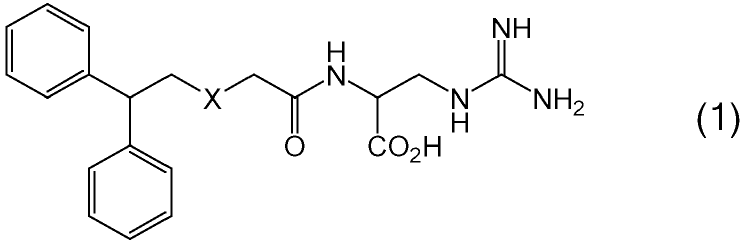 wherein X is O, S, CH₂ or NH, 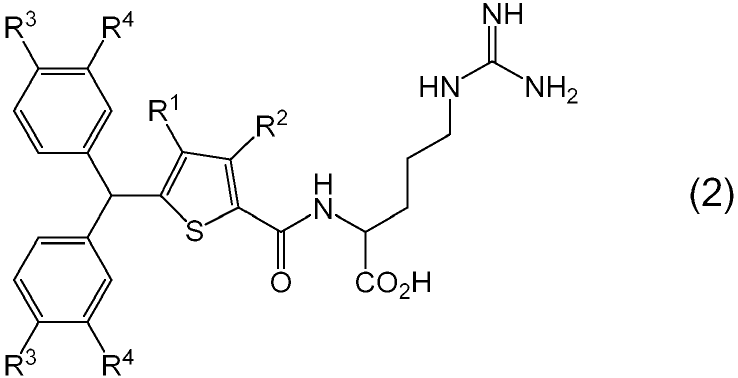 wherein R¹ is a hydrogen atom, a methyl group or a phenyl group, R² is a hydrogen atom, a methyl group or a bromine atom, R³ is a hydrogen atom, a hydroxy group, a methoxy group, a fluorine atom, or a chlorine atom, and R⁴ is a hydrogen atom or a chlorine atom.

5. A method for evaluating or selecting an agent for preventing or ameliorating intractable pruritus, comprising the following steps:
(1) evaluating an antagonistic effect of a test substance on C3a receptors, and
(2) evaluating or selecting the test substance having an antagonistic effect on C3a receptors as an agent for preventing or ameliorating intractable pruritus.

6. A method for evaluating or selecting an agent for preventing or ameliorating a pruritic skin disease presenting with intractable pruritus, comprising the following steps:
(1) evaluating an antagonistic effect of a test substance on C3a receptors, and
(2) evaluating or selecting the test substance having an antagonistic effect on C3a receptors as an agent for preventing or ameliorating a pruritic skin disease presenting with intractable pruritus.

7. Use of a C3a receptor antagonist for producing an agent for preventing or ameliorating intractable pruritus.

8. Use of a C3a receptor antagonist for producing an agent for preventing or ameliorating a pruritic skin disease presenting with intractable pruritus.

9. The use according to claim 8, wherein the pruritic skin disease presenting with intractable pruritus is atopic dermatitis or xeroderma.

10. The use according to any one of claims 7 to 9, wherein the C3a receptor antagonist is a compound of the following formula (1), a compound of the following formula (2), or a hexapeptide consisting of the sequence FLTChaAR (Cha: cyclohexylalanine), 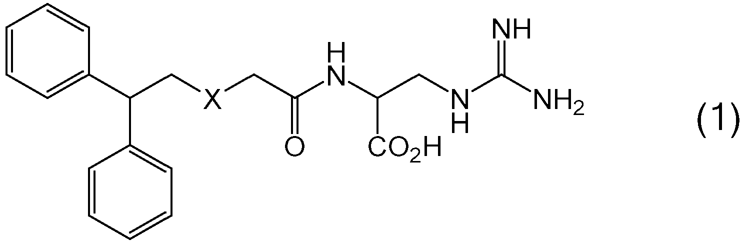 wherein X is O, S, CH₂ or NH, 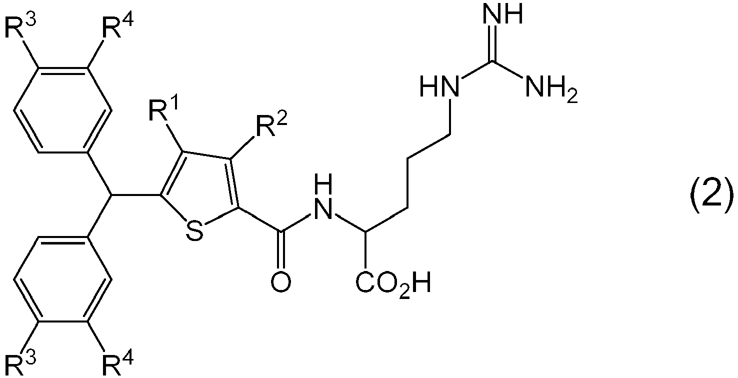 wherein R¹ is a hydrogen atom, a methyl group or a phenyl group, R² is a hydrogen atom, a methyl group or a bromine atom, R³ is a hydrogen atom, a hydroxy group, a methoxy group, a fluorine atom, or a chlorine atom, and R⁴ is a hydrogen atom or a chlorine atom.

11. A C3a receptor antagonist for use in the prevention or amelioration of intractable pruritus.

12. A C3a receptor antagonist for use in the prevention or amelioration of a pruritic skin disease presenting with intractable pruritus.

13. The C3a receptor antagonist according to claim 12, wherein the pruritic skin disease presenting with intractable pruritus is atopic dermatitis or xeroderma.

14. The C3a receptor antagonist according to any one of claims 11 to 13, which is a compound of the following formula (1), a compound of the following formula (2), or a hexapeptide consisting of the sequence FLTChaAR (Cha: cyclohexylalanine), 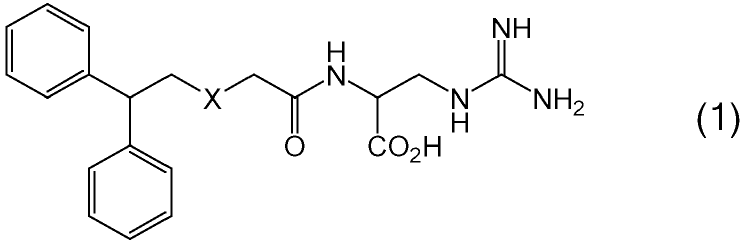 wherein X is O, S, CH₂ or NH, 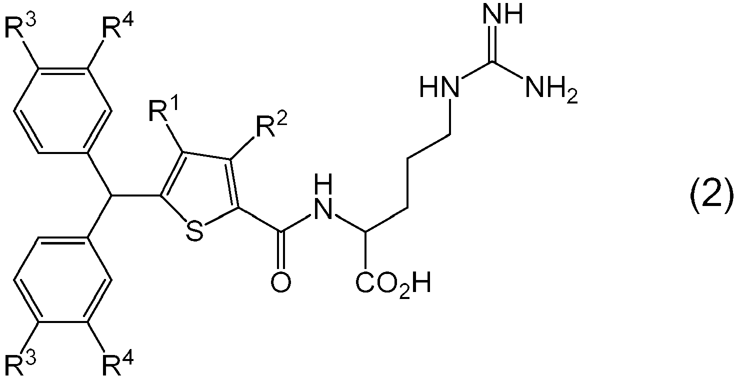 wherein R¹ is a hydrogen atom, a methyl group or a phenyl group, R² is a hydrogen atom, a methyl group or a bromine atom, R³ is a hydrogen atom, a hydroxy group, a methoxy group, a fluorine atom, or a chlorine atom, and R⁴ is a hydrogen atom or a chlorine atom.

15. Non-therapeutic use of a C3a receptor antagonist for preventing or ameliorating intractable pruritus.

16. Non-therapeutic use of a C3a receptor antagonist for preventing or ameliorating a pruritic skin disease presenting with intractable pruritus.

17. The non-therapeutic use according to claim 16, wherein the pruritic skin disease presenting with intractable pruritus is atopic dermatitis or xeroderma.

18. The non-therapeutic use according to any one of claims 15 to 17, wherein the C3a receptor antagonist is a compound of the following formula (1), a compound of the following formula (2), or a hexapeptide consisting of the sequence FLTChaAR (Cha: cyclohexylalanine), 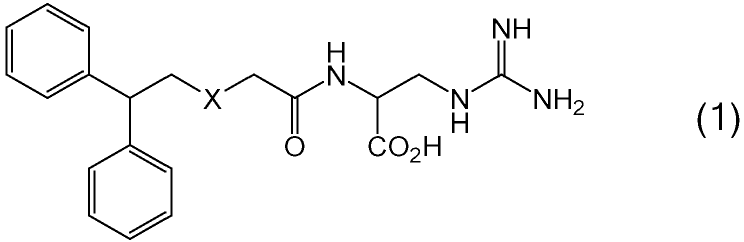 wherein X is O, S, CH₂ or NH, 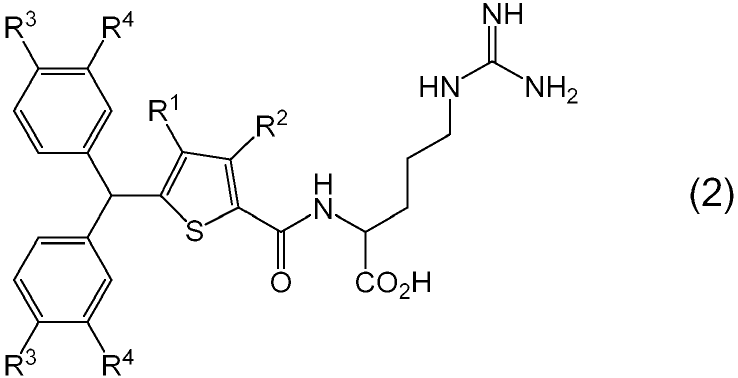 wherein R¹ is a hydrogen atom, a methyl group or a phenyl group, R² is a hydrogen atom, a methyl group or a bromine atom, R³ is a hydrogen atom, a hydroxy group, a methoxy group, a fluorine atom, or a chlorine atom, and R⁴ is a hydrogen atom or a chlorine atom.

19. A method for preventing or ameliorating intractable pruritus, comprising administering or applying an effective amount of C3a receptor antagonist to a subject in need thereof.

20. A method for preventing or ameliorating a pruritic skin disease presenting with intractable pruritus, comprising administering or applying an effective amount of C3a receptor antagonist to a subject in need thereof.

21. The method according to claim 20, wherein the pruritic skin disease presenting with intractable pruritus is atopic dermatitis or xeroderma.

22. The method according to any one of claims 19 to 21, wherein the C3a receptor antagonist is a compound of the following formula (1), a compound of the following formula (2), or a hexapeptide consisting of the sequence FLTChaAR (Cha: cyclohexylalanine), 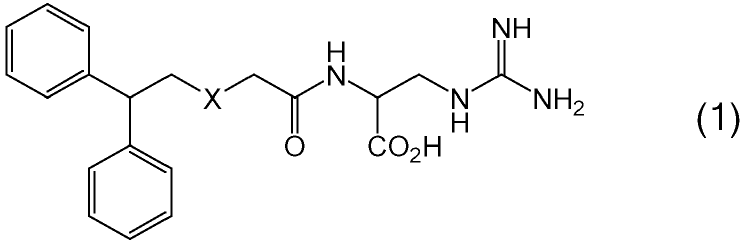 wherein X is O, S, CH₂ or NH, 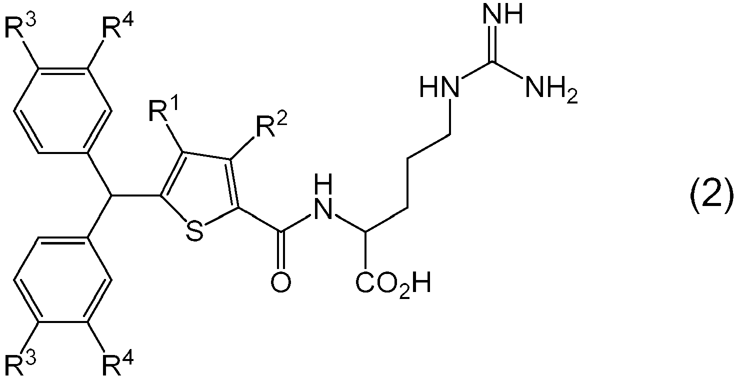 wherein R¹ is a hydrogen atom, a methyl group or a phenyl group, R² is a hydrogen atom, a methyl group or a bromine atom, R³ is a hydrogen atom, a hydroxy group, a methoxy group, a fluorine atom, or a chlorine atom, and R⁴ is a hydrogen atom or a chlorine atom.
